(19)

Europäisches Patentamt

European Patent Office

Office européen des brevets

(11) **EP 3 799 574 B1**

(12) **EUROPEAN PATENT SPECIFICATION**

(45) Date of publication and mention of the grant of the patent:
**01.01.2025 Bulletin 2025/01**

(21) Application number: **19713195.6**

(22) Date of filing: **12.02.2019**

(51) International Patent Classification (IPC):
*A61M 16/00* (2006.01)    *A61B 5/053* (2021.01)
*A61B 5/08* (2006.01)

(52) Cooperative Patent Classification (CPC):
**A61B 5/085; A61B 5/0536; A61B 5/087;**
**A61B 5/6823; A61B 5/6831; A61M 16/026;**
A61B 2562/0247; A61M 16/205; A61M 2016/0027;
A61M 2016/0036; A61M 2016/102; A61M 2205/50;
A61M 2230/46; A61M 2230/65

(86) International application number:
**PCT/IB2019/051091**

(87) International publication number:
**WO 2019/155438 (15.08.2019 Gazette 2019/33)**

(54) **SYSTEMS TO DETERMINE A PATIENT'S RESPONSIVENESS TO AN ALVEOLAR RECRUITMENT MANEUVER**

SYSTEME ZUR BESTIMMUNG DES ANSPRECHENS EINES PATIENTEN AUF EIN ALVEOLÄRES REKRUTIERUNGSMANÖVER

SYSTÈMES POUR DÉTERMINER LA RÉACTIVITÉ D'UN PATIENT À UNE MANOEUVRE DE RECRUTEMENT ALVÉOLAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.02.2018  US 201862629466 P**

(43) Date of publication of application:
**07.04.2021  Bulletin 2021/14**

(73) Proprietor: **TIMPEL MEDICAL B.V.**
**5656 AE Eindhoven (NL)**

(72) Inventor: **HOLZHACKER, Rafael**
**05417-020 Pinheiros,  São Paulo (BR)**

(74) Representative: **Studio Torta S.p.A.**
**Via Viotti, 9**
**10121 Torino (IT)**

(56) References cited:
**EP-A1- 2 228 009        US-A1- 2011 023 880**
**US-A1- 2012 010 520        US-A1- 2015 290 418**
**US-B1- 6 390 092**

• **LONG YUN ET AL: "Assessment of Lung Recruitment by Electrical Impedance Tomography and Oxygenation in ARDS Patients :", MEDICINE., vol. 95, no. 22, 1 May 2016 (2016-05-01), US, pages e3820, XP055587573, ISSN: 0025-7974, DOI: 10.1097/ MD.0000000000003820**

**Description**

TECHNICAL FIELD

The disclosure relates to systems and method for determining a patient's potential for recruitment of the patient's lung capacity through an alveolar recruitment maneuver. In particular, this disclosure relates to systems and methods that include and utilize an electrical impedance tomography (EIT) system in determining a patient's potential for recruitment.

BACKGROUND

The treatment of acute respiratory distress syndrome ("ARDS") includes a proper mechanical ventilation strategy. The alveolar recruitment maneuver ("ARM") is an intervention applied in moderate and severe cases of ARDS. ARM is a transitory and controlled increase in mechanical ventilator pressure delivered to the lungs aiming to open previously collapsed alveoli. However, some patients respond well to an ARM maneuver (referred to herein as "responders"), while other patients do not respond well to the ARM maneuver (referred to herein as "non-responders"). Caregivers should assess a patient's responsiveness to an ARM maneuver, and apply it only on the patients that will benefit from the ARM maneuver (responders). The ARM maneuver may also introduce some additional risks, such as Ventilatory Induced Lung Injury (VILI) and hemodynamic impairment. Conventional methods for determining whether the patient is responsive to an ARM maneuver include Computed Tomography (CT) and Pulmonary Mechanics.

CT may be used as a method to estimate amount of lung collapse (e.g., in grams and/or in percentage of lung weight), and the amount of lung that was reopened. However, it provides only static images, it requires the use of excessive radiation (it is necessary to scan the whole lung at two different PEEP levels - at least), and it requires long ins/expiratory pauses (with risks of hypercapnia and hemodynamic impairment). Finally, it is necessary to transport the patient from the ICU to the radiology department, with well known risks.

Pulmonary Mechanics may be used as a method to determine a patient's responsiveness using positive and expiratory pressure ("PEEP") therapy with a mechanical ventilator as described in U.S. Patent Application Publication No. 2012/0010520. The patient is provided PEEP therapy at a first PEEP. Lung compliance and a first end expiratory lung volume (EELV) is measured from the patient, with the help of an accessory technique like CT or nitrogen washout. PEEP therapy is provided to the patient at a second PEEP. A second EELV is measured from the patient. A difference from the first EELV and the second EELV measured may be calculated, as well as the "predicted-change" in EELV if assuming no lung recruitment from one PEEP step to the next. An index indicative of the patient's response to PEEP therapy is calculated from the difference between the first EELV and the second EELV, after accounting the "predicted-change" in FRC. This index is expressed in volume units, or as a ratio between this "above-predicted" volume and the functional residual capacity (FRC), or still as the ratio between the "above-predicted" volume and the compliance at the first PEEP step. This index may be used to differentiate high recruiters from low recruiters.

European Patent Publication 2 228 009 describes an apparatus and a method for determining functional lung characteristics of a patient utilising an electrical impedance tomography (EIT) imaging device adapted to record the impedance distribution within a plane of the thorax of the patient.

SUMMARY

The present invention relates to a system for determining a potential lung recruitment value for a patient, as defined in the annexed claim 1. Preferred embodiments are matter of the dependent claims.

DISCLOSURE

Some embodiments of the present disclosure include methods for determining a potential lung recruitment value for a patient. The methods are however not separately claimed. The methods may include during an applied first positive end expiratory pressure to at least one lung of the patient, measuring a first end expiratory lung impedance of the at least one lung of the patient at the first positive end expiratory pressure; during an applied second positive end expiratory pressure to the at least one lung of the patient, measuring a second end expiratory lung impedance of the at least one lung of the patient at the second positive end expiratory pressure; determining a change in end expiratory lung impedance between the positive end expiratory pressure and the second positive end expiratory pressure; determining a first chord-compliance of the at least one lung of the patient from pixels of a first electrical impedance tomography image of the patient at the first positive end expiratory pressure; determining a second chord-compliance of the at least one lung of the patient from pixels of a second electrical impedance tomography image of the patient at the second positive end expiratory pressure; determining a first index representing the change in end expiratory lung impedance based on at least one of the first chord-compliance or second chord-compliance; determining a second index representing a change in compliance;

and based on the first index and the second index, determining a potential lung recruitment value for the patient.

One or more embodiments of the present disclosure may include a system for determining a potential lung recruitment value for a patient. The system may include at least one processor and at least one non-transitory computer readable storage medium storing instructions thereon that, when executed by the at least one processor, cause the at least one processor to: in response to a sequence of positive end expiratory pressures being applied to lungs of a patient, cause an end expiratory lung impedance to be measured at each positive end expiratory pressure of the sequence of positive end expiratory pressures; determine a first index representing a change in end expiratory lung impedance between a given end expiratory lung volume of the sequence of positive end expiratory pressures and a subsequent end expiratory lung volume of the sequence of positive end expiratory pressures; determine a second index representing change in chord-compliance of the lungs of the patient between the given end expiratory lung volume and the subsequent end expiratory lung volume; and based on the first index and the second index, determine a potential lung recruitment value for the patient.

One or more embodiments of the present disclosure may include a system for determining a potential lung recruitment value for a patient. The system may include a ventilator system, an electrical impedance tomography system, and a controller, wherein the ventilator system and the electrical impedance tomography system are operably coupled to the controller. The controller may include at least one processor and at least one non-transitory computer readable storage medium storing instructions thereon that, when executed by the at least one processor, cause the at least one processor to: cause the ventilator system to apply a sequence of positive end expiratory pressures to the lungs of a patient; cause the ventilator system to measure an end expiratory lung impedance at each positive end expiratory pressure of the sequence of positive end expiratory pressures; determine a first index representing a change in end expiratory lung impedance between at least two applied positive end expiratory pressures; determine a second index representing a change in overall compliance of the lungs of the patient between the at least two applied positive end expiratory pressures based on impedance measurements represented within electrical impedance tomography images generated by the electrical impedance tomography system; and based on the first index and the second index, determine a potential lung recruitment value for the patient.

BRIEF DESCRIPTION OF THE DRAWINGS

FIG. 1 is a schematic diagram of a portion of an EIT system showing a plurality of electrodes positioned around a region of interest of a patient according to one or more embodiments of the present disclosure;

FIG. 2 is a schematic diagram showing a cross-section of the thorax of the patient along the plane of the electrodes according to one or more embodiments of the present disclosure;

FIG. 3 is a schematic block diagram of an EIT system according to an embodiment of the disclosure;

FIG. 4 shows a schematic view of system for determining a potential recruitment value of a patient's lungs according to one or more embodiments of the present disclosure;

FIG. 5 is a flow chart of a method of determining a potential recruitment value of a patient according to one or more non-claimed embodiments of the present disclosure;

FIG. 6A shows a graph of applied and/or measured air pressures over time representing a sequence of PEEPs applied to the lungs of a patient according to one or more embodiments of the present disclosure;

FIG. 6B shows a graph representing an EIT plethysmograph measured over time and collected simultaneously to the airway pressures of FIG. 6A;

FIG. 7 shows a graph of the lung volume of the patient calculated from a EIT plethysmogram plotted against a calculated alveolar pressure;

FIG. 8 shows exponential pressure-volume curves representing a simulated lung inflation when all lung units are already recruited at the start of inflation;

FIG. 9 shows a graph of the simulated lung inflation of FIG. 8;

FIG. 10 shows a graph of calculated chord compliances of lungs plotted against applied PEEPs, using the same simulated lung model represented in FIGS. 8 and 9;

FIG. 11 shows simulated pressure-volume relationships during lung inflation when modeling a lung with multiple compartments;

FIG 12 shows a function defining the probability or frequency of occurrence of recruitment of new lung units when lung inflation progresses;

FIGS. 13 and 14 show graphs representing two different individuals, a first with a normal lung and very small recruitable lung tissue compared with a second who had lung injury and has a relatively large potential for recruitment; and

FIG. 15 shows EIT images of three patients collected simultaneously where the first index is represented in the left column of images and the second index is represented in the right column of images.

# EP 3 799 574 B1

## MODE(S) FOR CARRYING OUT THE INVENTION

In the following detailed description, reference is made to the accompanying drawings which form a part hereof, and in which is shown by way of illustration specific embodiments in which the disclosure may be practiced. These embodiments are described in sufficient detail to enable those of ordinary skill in the art to practice the disclosure. It should be understood, however, that the detailed description and the specific examples, while indicating examples of embodiments of the disclosure, are given by way of illustration only and not by way of limitation. From this disclosure, various substitutions, modifications, additions rearrangements, or combinations thereof within the scope of the disclosure may be made and will become apparent to those of ordinary skill in the art.

In accordance with common practice, the various features illustrated in the drawings may not be drawn to scale. The illustrations presented herein are not meant to be actual views of any particular apparatus (e.g., device, system, etc.) or method, but are merely representations that are employed to describe various embodiments of the disclosure. Accordingly, the dimensions of the various features may be arbitrarily expanded or reduced for clarity. In addition, some of the drawings may be simplified for clarity. Thus, the drawings may not depict all of the components of a given apparatus or all operations of a particular method.

Information and signals described herein may be represented using any of a variety of different technologies and techniques. For example, data, instructions, commands, information, signals, bits, symbols, and chips that may be referenced throughout the description may be represented by voltages, currents, electromagnetic waves, magnetic fields or particles, optical fields or particles, or any combination thereof. Some drawings may illustrate signals as a single signal for clarity of presentation and description. It should be understood by a person of ordinary skill in the art that the signal may represent a bus of signals, wherein the bus may have a variety of bit widths and the disclosure may be implemented on any number of data signals including a single data signal.

The various illustrative logical blocks, modules, and circuits described in connection with the embodiments disclosed herein may be implemented or performed with a general purpose processor, a special purpose processor, a Digital Signal Processor (DSP), an Application Specific Integrated Circuit (ASIC), a Field Programmable Gate Array (FPGA) or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. A general-purpose processor may be considered a special-purpose processor while the general-purpose processor executes instructions (e.g., software code) stored on a computer-readable medium. A processor may also be implemented as a combination of computing devices, e.g., a combination of a DSP and a microprocessor, a plurality of microprocessors, one or more microprocessors in conjunction with a DSP core, or any other such configuration.

Also, it is noted that embodiments may be described in terms of a process that may be depicted as a flowchart, a flow diagram, a structure diagram, or a block diagram. Although a flowchart may describe operational acts as a sequential process, many of these acts can be performed in another sequence, in parallel, or substantially concurrently. In addition, the order of the acts may be re-arranged. A process may correspond to a method, a function, a procedure, a subroutine, a subprogram, etc. Furthermore, the methods disclosed herein may be implemented in hardware, software, or both. If implemented in software, the functions may be stored or transmitted as one or more instructions or code on computer-readable media. Computer-readable media include both computer storage media and communication media, including any medium that facilitates transfer of a computer program from one place to another.

As used herein, the singular forms following "a," "an," and "the" are intended to include the plural forms as well, unless the context clearly indicates otherwise.

As used herein, the term "may" with respect to a material, structure, feature, or method act indicates that such is contemplated for use in implementation of an embodiment of the disclosure, and such term is used in preference to the more restrictive term "is" so as to avoid any implication that other compatible materials, structures, features, and methods usable in combination therewith should or must be excluded.

It should be understood that any reference to an element herein using a designation such as "first," "second," and so forth does not limit the quantity or order of those elements, unless such limitation is explicitly stated. Rather, these designations may be used herein as a convenient method of distinguishing between two or more elements or instances of an element. Thus, a reference to first and second elements does not mean that only two elements may be employed there or that the first element must precede the second element in some manner. Also, unless stated otherwise a set of elements may comprise one or more elements.

As used herein, the term "substantially" in reference to a given parameter, property, or condition means and includes to a degree that one skilled in the art would understand that the given parameter, property, or condition is met with a small degree of variance, such as within acceptable manufacturing tolerances. For example, a parameter that is substantially met may be at least about 90% met, at least about 95% met, or even at least about 99% met.

As used herein, the term "about" used in reference to a given parameter is inclusive of the stated value and has the meaning dictated by the context (e.g., it includes the degree of error associated with measurement of the given parameter, as well as variations resulting from manufacturing tolerances, etc.).

Embodiments of the disclosure include an electrical impedance tomography (EIT) device configured to determine the potential for recruitment responsive to calculating a the potential of the total lung mass - equivalent to the percentage of the total number of lung alveoli - that would be effectively recruited during an alveolar recruitment maneuver ("ARM"). For any given ARM, a threshold for this percentage (e.g., 20%) may be established that is used to differentiate high recruiters from low recruiters. As used herein, the term "recruit" and any derivative terms when used in reference to alveoli, parenchyma, and/or lungs refers to opening previously collapsed alveoli within the parenchyma of a lung or a portion of a lung such that the alveoli generally remain open.

EIT is an imaging technique involving the positioning electrodes via an electrode belt placed around a region of a patient's body (e.g., around the patient's chest for imaging of a lung), injecting electrical excitation signals through a pair of electrodes, and measuring the induced response signals detected by the other electrodes of the electrode belt. As a result, the EIT system may generate an image based on the voltage measurements indicating estimated impedance values. In contrast with other imaging techniques, EIT is non-invasive and does not have certain exposure risks that might limit the number and frequency of monitoring actions (e.g., as with techniques such as X-rays). As a result, EIT is suitable for continuously monitoring the condition of the patient, with particular application to monitoring the patient's lungs as the measurements may be used to determine respiratory and hemodynamic parameters of the patient and monitor a real-time two-dimensional image.

Some embodiments of the disclosure include a method (which is not part of the claimed invention), an apparatus, and a system for determining a patient's responsiveness to an ARM. Embodiments of the disclosure may be implemented by Electrical Impedance Tomography (EIT), or by a combination of information provided by EIT and flow and/or pressure sensors (e.g., pulmonary mechanics using gas monitoring sensors). Embodiments may better account for the components that are due to the stretching of already-aerated lung, versus due to actual recruitment of previously collapsed alveoli, providing accurate quantification of recruitment. Embodiments may also be implemented during tidal breaths, without the need of any especial maneuver except a sequence of different PEEP levels. In some embodiments, information at the pixel level in EIT images (e.g., the location of the pixel, the pixel aeration changes, and the pixel compliance changes during a recruitability assessment maneuver ("RAM")) may be combined to produce more accurate and regional quantification. The device may be configured to determine the spatial location of recruitment within an EIT-cross-sectional image and/or EIT-3D image.

Some embodiments of the present disclosure include a system for determining s "potential for lung recruitment" value. The system may include a ventilator system, an EIT system, and a controller. The controller may cause the ventilator system to apply a sequence of PEEPs to lungs (or to automatically sense a manual change in PEEP), and causes the EIT system to measure one or more indexes simultaneously. A first index represents a change of EELV for each pixel, or each lung region represented in a dynamic EIT image for each PEEP of the sequence of PEEPs. The controller may further automatically calculate the increase of EELV that is "above predicted" (e.g., above a predicted value) based on pressure-volume relationships observed during the previous PEEP step. A second index represents a change in pixel compliance or the change in regional compliance of multiple regions of the lung, and the calculation of the change in pixel compliance that is "above predicted" based on the pressure difference between two PEEP steps and based on algorithms describing the elastic lung behavior. A weighted composition of two or more indexes, or just one of them, may be determined to determine the potential for lung recruitment of that patient, and to classify the patient as responder or non-responder. In order to improve the classification, the indexes may be normalized by equations of predicted lung volumes such as vital capacity, total lung capacity, residual capacity, or inspiratory capacity obtained from human population studies, and/or from anthropometric measurements of the patient.

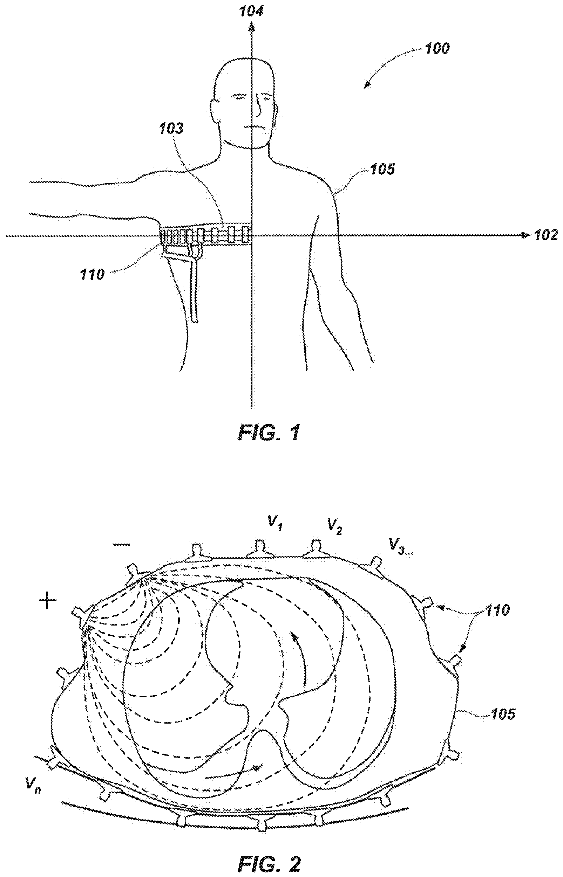

FIG. 1 is a schematic diagram of a portion of an EIT system 100 showing a plurality of electrodes 110 positioned around a region of interest (e.g., thorax) of a patient 105. The electrodes 110 of conventional EIT systems 100 are typically physically held in place by an electrode belt 103. The placement of the electrodes 110 is typically transverse to the cranial caudal axis 104 of the patient. Although the electrodes 110 are shown in FIG. 1 as being placed only partially around the patient 105, electrodes 110 may by placed around the entire patient 105 depending on the specific region of interest available or desired for measurement. The electrodes 110 may be coupled to a computing system (not shown) configured to control the operation of the electrodes 110 and perform reconstruction of the EIT image.

FIG. 2 is a schematic diagram showing a cross-section of the thorax of the patient 105 along the plane of the electrodes. A voltage may be applied to a pair of electrodes 110 (shown by the electrodes having a + and - symbol) to inject an excitation current into the patient between an electrode pair. As a result, voltages (e.g., $V_1, V_2, V_3 ... V_n$) may be detected by the other electrodes and measured by the EIT system 100. Current injection may be performed for a measurement cycle according to a circular pattern using different electrode pairs to generate the excitation current.

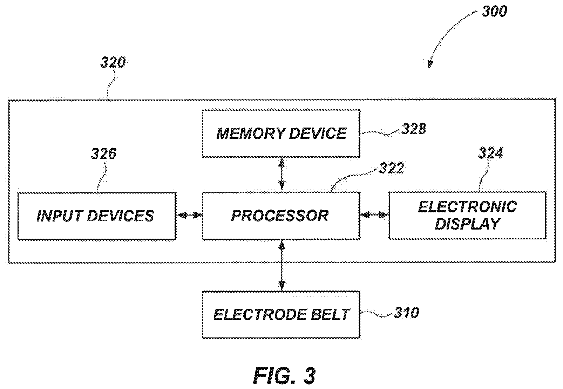

FIG. 3 is a schematic block diagram of an EIT system 300 according to an embodiment of the disclosure. The EIT system 300 may include an electrode belt 310 operably coupled with a data processing system 320. The electrode belt 310 and the data processing system 320 may be coupled together via a wired connection (e.g., cables) and/or may have communication modules to communicate wirelessly with each other. The data processing system 320 may include a processor 322 operably coupled with an electronic display 324, input devices 326, and a memory device 328. The electronic display 324 may be constructed with the data processing system 320 into a singular form factor for an EIT device coupled with the electrode belt 310. In some embodiments, the electronic display 324 and the data processing system 320 may be separate units of the EIT device coupled with the electrode belt 310. In yet other embodiments, an EIT system 300 may be integrated within another host system configured to perform additional medical measurements and/or procedures, in which the electrode belt 310 may couple to a port of the host system already having its own input devices, memory devices, and electronic display. As such, the host system may have the EIT processing software installed therein. Such software may be built into the host system prior field use or updated after installation.

The processor 322 may coordinate the communication between the various devices as well as execute instructions stored in computer-readable media of the memory device 328 to direct current excitation, data acquisition, data analysis, and/or image reconstruction. As an example, the memory device 328 may include a library of finite element meshes used by the processor 322 to model the patient's body in the region of interest for performing image reconstruction. Input devices 326 may include devices such as a keyboard, touch screen interface, computer mouse, remote control, mobile devices, or other devices that are configured to receive information that may be used by the processor 322 to receive inputs from an operator of the EIT system 300. Thus, for a touch screen interface the electronic display 324 and the input devices 326 receiving user input may be integrated within the same device. The electronic display 324 may be configured to receive the data and output the EIT image reconstructed by the processor for the operator to view. Additional data (e.g., numeric data, graphs, trend information, and other information deemed useful for the operator) may also be generated by the processor 322 from the measured EIT data alone, or in combination with other non-EIT data according to other equipment coupled thereto. Such additional data may be displayed on the electronic display 324.

The EIT system 300 may include components that are not shown in the figures, but may also be included to facilitate communication and/or current excitation with the electrode belt 310 as would be understood by one of ordinary skill in the art, such as including one or more analog to digital converter, signal treatment circuits, demodulation circuits, power sources, etc.

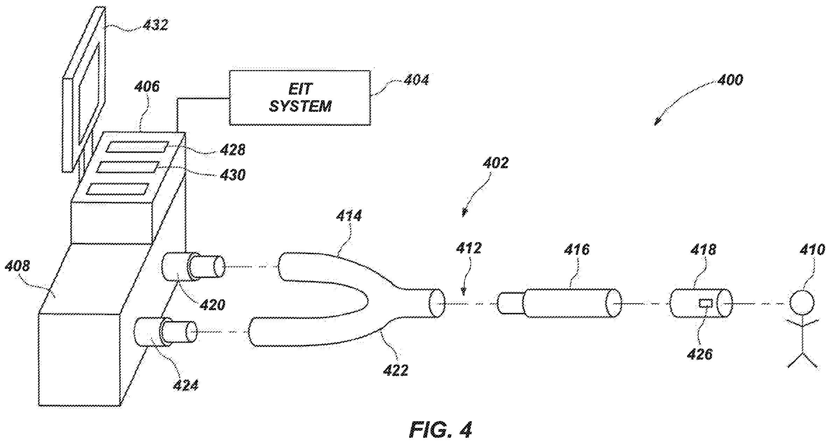

FIG. 4 depicts a schematic view of system 400 for determining a patient's a potential for recruitment of a patients' lung capacity (e.g., collapsed parenchyma) during an alveolar recruitment maneuver ("ARM"). For instance, FIG. 4 depicts a system 400 for determining if a given patient is a good candidate for an ARM (e.g., has a relatively high recruitment potential) or is a poor candidate for an ARM (e.g., has a relatively low recruitment potential). Referring to FIG. 4, the system 400 may include a ventilator system 402, an EIT system 404, and a controller 406 for operating the system 400 and specifically, the ventilator system 402, an EIT system 404. The ventilator system 402 and the EIT system 404 are operably coupled to the controller 406.

In some embodiments, the ventilator system 402 includes a mechanical ventilator 408 that provides respiratory support or respiratory assistance to a patient 410. For instance, the mechanical ventilator 408 may provide a flow of medical gas, which may include one or more of air, oxygen, nitrogen, and helium. In some embodiments, the flow of medical gas may further include additives such as aerosol drugs or anesthetic agents. The ventilator system 402 may further include a breathing circuit 412, an inspiratory limb 414, a patient limb 416, and a patient connection 418. In some embodiments, the mechanical ventilator 408 may provide a flow of medical gas to the breathing circuit 412 through the inspiratory limb 414, which is connected to an inspiratory port 420 of the mechanical ventilator 408. The medical gas may flow (e.g., travel) through the inspiratory limb 414 and into the patient limb 416 of the breathing circuit 412. Accordingly, the mechanical ventilator 408 may provide thee medical gas to the patient 410 through the patient connection 418.

Expired gases from the patient 410 may be delivered back to the mechanical ventilator 408 through the patient connection 418 and the patient limb 416. In some embodiments, the expired gases may be directed into an expiratory limb 422 of the breathing circuit 412 via one or more valves (e.g., check valves). For instance, the ventilator system 402 may further include a plurality of check valves,) which may be placed at various points along the breathing circuit 412 such as to only permit medical gas flow in a desired direction along the appropriate pathway towards or away from the patient 410.

Additionally, the expired gases may be returned to the mechanical ventilator 408 through an expiratory port 424 of the mechanical ventilator 408.

In some embodiments, the expiratory port 424 may include a controllable flow valve that is adjustable to regulate the pressure within the breathing circuit 412. Adjusting the flow valve may create a back pressure, which is applied to the patient 410 during exhalation to create a positive end expiratory pressure ("PEEP"). Accordingly, the system 400 may include any conventional system for providing PEEP therapy to a patient 410. Additionally, other systems and configurations as recognized by one of ordinary skill in the art fall within the scope of the present disclosure.

The ventilator system 402 may further include one or more gas monitoring sensors 426. In some embodiments, the one or more gas monitoring sensors 426 may be disposed within the patient connection 418 of the breathing circuit 412. In alternative embodiments, the one or more gas monitoring sensors 426 may be fluidly connected to any other component of the breathing circuit 412 or the ventilator system 402. In some embodiments, the gas monitoring sensor 426 may include one or more of a pressure, a flow, and a gas concentration sensor. As is described in further detail below, the controller 406 and the mechanical ventilator 408 may utilize the one or more gas monitoring sensors 426 to monitor and ultimately, control the operation of the mechanical ventilator 408 and provide information (e.g., feedback to a user (e.g., clinician)). In some embodiments, the one or more gas sensors 426 may include any conventional gas sensors.

The EIT system 404 may include any of the EIT systems described above in regard to FIGS. 1-3 and may operate according to any of the embodiments described above. In some embodiments, the EIT system 404 may include any conventional EIT system. Additionally, as noted above, the EIT system 404 may be operably coupled to the controller 406 and may provide information to controller 406 regarding measurements performed by the EIT system 404. In some embodiments, the EIT system 404 may be completely independent of the ventilator system 402 and may determine that the PEEP was changed during a maneuver through via a respective pressure sensor operably coupled to the EIT system 404. In one or more embodiments, the EIT system 404 may also have a respective electronic display and input devices separate from displays and/or input devices of the ventilator system 402. Furthermore, the electronic display and input devices of the EIT system 404 may be utilized to input (e.g., manually input) information about the PEEP and/or other ventilatory parameters.

The controller 406 may include a processor 428 coupled to a memory 430 and an input/output component 432. The processor 428 may comprise a microprocessor, a field-programmable gate array, and/or other suitable logic devices. The memory 430 may include volatile and/or nonvolatile media (e.g., ROM, RAM, magnetic disk storage media, optical storage media, flash memory devices, and/or other suitable storage media) and/or other types of computer-readable storage media configured to store data. The memory 430 may store algorithms and/or instructions for operating the ventilator system 402 and the EIT system 404, to be executed by the processor 428. For example, the controller 406 may include the data processing system 320 described above in regard to FIG. 3. In some embodiments, the processor 428 is operably coupled to send data to a computing device operatively coupled (e.g., over the Internet) to the controller 406, such as a server or personal computer. The input/output component 432 can include a display, a touch screen, a keyboard, a mouse, and/or other suitable types of input/output devices configured to accept input from and provide output to an operator.

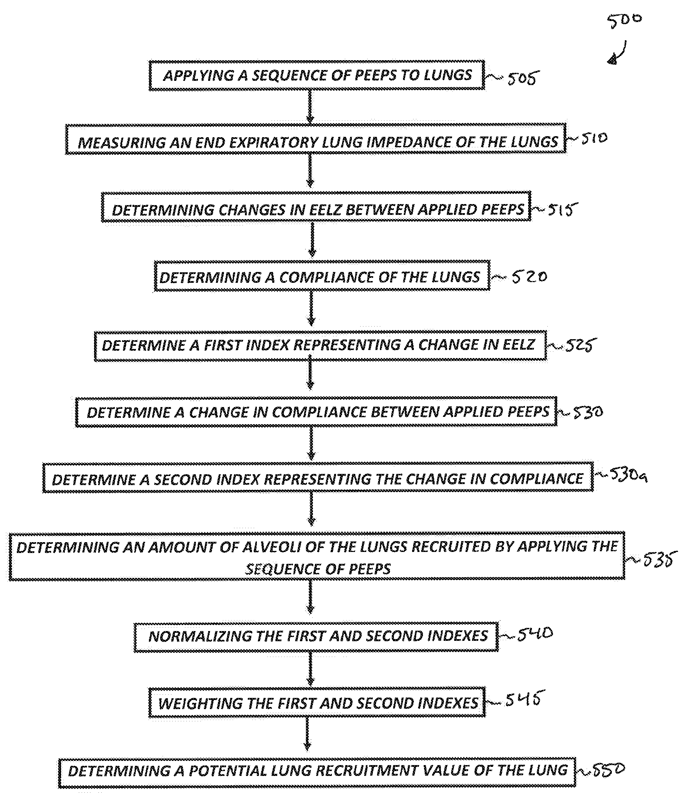

Referring still to FIG. 4, as is described in greater detail below in regard to FIGS. 5-13, the system 400 may utilize the ventilator system 402 to apply levels of PEEP to a patient in determining a potential for recruitment value of a patients' lung capacity (e.g., collapsed parenchyma) during an RAM. PEEP increases a base line pressure within the patient's respiratory system such that natural exhalation by the patient maintains a higher airway pressure than respiration without PEEP therapy. Conventional PEEP pressures range up to 40 cm H2O, although higher PEEP pressures may also be used. High PEEP refers to PEEP therapy applied above 10 cm H2O, and more specifically, 10-30 $cmH_2O$. Low PEEP refers to PEEP pressures below 10 cm H2O and which are often applied at 5-8 cm H2O.

In some embodiments, the effects of applying PEEP to a patient are measured by measuring a volume of the patient's lungs in response to the application of PEEP. After PEEP application, the volume of the patient's lungs is measured as the end expiratory lung volume (EELV) and is measured for a particular PEEP pressure applied to the patient. In some embodiments, EELV is measured at zero PEEP ("ZEEP"). The measurement of EELV at ZEEP is referred to herein as functional residual capacity (FRC) and is a measurement of the volume of air that remains in the lungs at the end of natural expiration. In some embodiment, when utilizing the EIT system 404 to measure and/or determine EELV, the FRC can, in some instances, be considered to be zero such that the FRC does not affect certain calculations. In view of the foregoing, EELV is FRC plus lung volume increased by the applied PEEP.

Increases in EELV associated with the application of PEEP come from two physiological sources. A first physiological source of volume increase results from the application of additional pressure on the lung tissue. Applying additional pressure on lung tissue causes the lungs and already-opened alveoli to distend (e.g., swell due to pressure inside the lungs), creating more lung volume. Distending the lungs presents risks to a patient in the form of volutrauma (i.e., local over distention of normal alveoli), which damages the lungs. Volutrauma can result in medical complications with the patient similar to Acute Respiratory Distress Syndrome (ARDS). A second physiological source of increased lung volume is the "recruitment" of alveoli in a process known as "pop-open," where an internal volume of one alveolus suddenly jumps from a zero volume (e.g., collapsed) to a volume attained by neighboring alveoli (e.g., neighboring units). As is known in the art, alveoli are the air sacs within the lungs that promote gas exchange with a patient's blood. Some alveoli, particularly diseased or distressed alveoli, collapse when the pressure within the lungs falls too low, with the alveoli (e.g., unit) attaining or reaching a zero volume. Applying PEEP to a patient (e.g., applying a PEEP therapy) can maintain a minimum airway pressure within the lungs and, in some instances, cause alveoli (e.g., collapsed alveoli) to remain open.

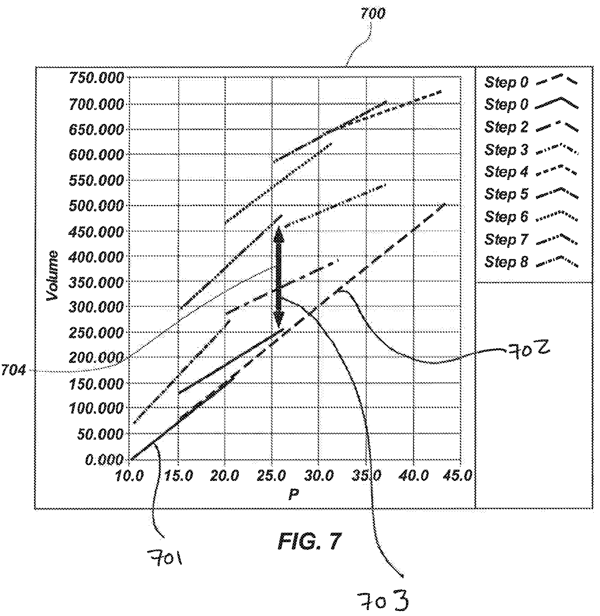

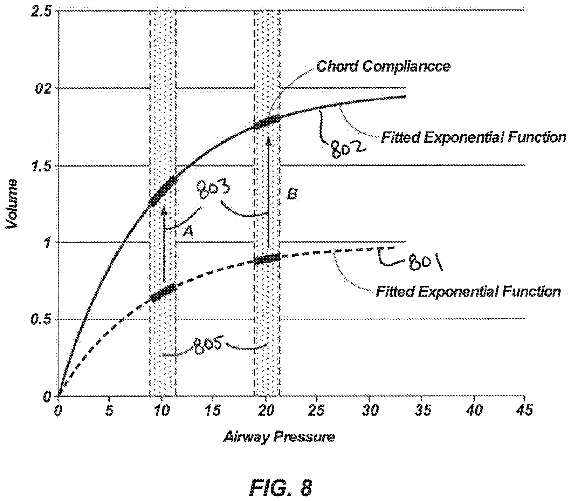

The alveoli can only generate some gas exchange when the alveoli are open. Therefore, in order to maintain some gas exchange when PEEP is insufficient, there is a need of higher driving inspiratory pressure to hyperventilate the already opened alveoli in order to compensate for the lack of function of the closed alveoli (e.g., units). Increased respiratory force causes greater distension on the remaining open alveoli, which may result in further damage to the lung tissue. In contrast, when PEEP is sufficient, the gas exchange is shared among most alveoli (e.g., units), which decreases the driving inspiratory pressure and, in some instances, lung damage. The recruitment of alveoli, therefore, also increases EELV, which correspond to the extra-volume generated by the pop-open of multiple alveolar units. The extra-volume created by opened alveolar units is known as the volume-gain (or vertical displacement in a pressure-volume plot) of the lung at a certain pressure. In a graph having two pressure-volume plots representing lung inflation with a first plot representing the inflation of the lung in a case of no recruitment, and with a second plot representing the conditional inflation of the lung when lung alveoli (e.g., units) were opened since a beginning of inflation, a vertical distance between the two curves (see FIGS. 7 and 8) represents a volume-gain caused by recruitment.

FIG. 5 is a flow chart of a method 500 of determining a potential lung recruitment value (referred to hereinafter a "potential recruitment value" or "PRC"). As used herein, the phrase "potential recruitment value" may refer to percentage of a total lung mass (or volume) that is determined to be effectively recruitable during an ARM. As is be described herein, the PRC is determined using both the EIT system 404 and the ventilator system 408, as depicted in FIGS. 1-4.

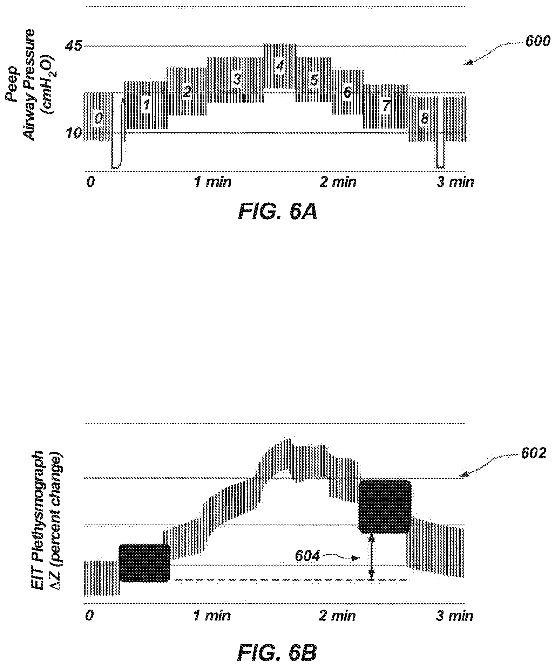

In some non-claimed embodiments, the method may include applying a sequence of PEEPs to the lungs (e.g., at least one lung) of a patient, as shown in act 505 of FIG. 5. FIGS. 6A and 6B are referred to in describing act 505 of FIG. 5. FIG. 6A shows a graph 600 representing a sequence of PEEPs applied to and/or measured from the lungs of a patient over time, and FIG. 6B shows a graph 602 representing an EIT plethysmograph measured over time. The EIT plethysmograph of FIG. 6B was collected simultaneously to the airway pressures of FIG. 6A, during the ascending and descending PEEP steps utilized to assess recruitability of alveoli and/or the potential for lung recruitment. In particular, as is discussed in greater detail below, the plethysmograph represents a sum of the underlying impedance of pixels of an entire EIT image or a certain region of interest of the lungs within an EIT image. In some embodiments, the PEEPs may be determined (e.g., measured or collected) from a pressure sensor proximate to an entrance of the endotracheal tube of the patient. The graphs 600, 602 of FIGS. 6A and 6B may be constructed from each pixel within the EIT image. In some embodiments, the graphs 600, 602 may represent a certain region of interest of a lung and/or lungs. In other embodiments, the graphs 600, 602 may represent the entire lung. Furthermore, each pixel of the EIT image results in a particular plethysmogram because of the particular properties of the region of the lungs represented by the pixel.

Referring to FIGS. 5-6B together, in one or more embodiments, applying a sequence of PEEPs to the lungs of a patient may include applying a plurality of sequential PEEPs to the patient where the PEEPs increase or decrease relative to a previously applied PEEP. For instance, in the example depicted in FIGS. 6A and 6B, an amount of PEEP was increased for four steps (e.g., steps 1-4) (e.g., four increasing levels of PEEP were applied) followed by four steps of decreasing amounts of PEEP (i.e., decremental PEEPs). As is discussed in greater detail below, applying at last one level of decremental PEEP enables a better estimate of a factor K, which is utilized in determining predicted-compliance values of the lungs or a predicted-volume values of the lungs at a future PEEP level for the patient. In the example depicted in FIGS. 6A and 6B, PEEP was increased from about 10 cmH$_2$O to about 30 cmH$_2$O through steps 1-4, and then the PEEP was deceased stepwise back to 10 cmH$_2$O through steps 5-8. The sequence of PEEPs may be applied via any of the manners described above in regard to FIG. 4 and ventilator system 408 of the system 400. Additionally, the sequence of PEEPs may be applied manually, with the controller 406 (FIG. 4) or the EIT system 404 detecting changes in PEEP automatically. Thus, the sequence of PEEPs may be applied via any conventional manner known in the art for applying PEEP therapy.

In some embodiments, a first PEEP of the sequence of PEEPs may be different from a second PEEP of the sequence of PEEPs. Furthermore, in one or more embodiments, the second PEEP may be an increased PEEP relative to the first PEEP, and a third subsequent PEEP may be a decreased PEEP relative to the second PEEP.

In one or more non-claimed embodiments, the method 500 may further include measuring an end expiratory lung impedance ("EELZ") of the lungs of the patient at each PEEP of the sequence of PEEPs, as shown in act 510 of FIG. 5. In some embodiments, measuring an EELZ of the lungs of the patient at a given PEEP may include determining an impedance value represented in EIT images of the lungs of the patient. Furthermore, the EELZ may be representative of an end expiratory lung ("EELV"). For instance, the EELV of the lungs may be determined based on (e.g., from) the measured EELZ of the lungs. As will be appreciated in view of the present disclosure, in some embodiments, act 510 may include determining an EELZ of just a region (e.g., at least one region, a region of interest, etc.) of a lung of the patient. In some embodiments, act 510 may further include directly determining and/or measuring an EELV of the lungs of the patient at a given PEEP of the sequence of PEEPs via the one or more gas monitoring sensors 426 described above in regard to FIG. 4. For instance, the one or more gas monitoring sensors 426 may include one or more of a volumetric sensor, a flow sensor, a pressure sensor, a concentration sensor, or any combination thereof. Furthermore, the EELV may be measured and/or determined via a variety of methods including body plethysmography, helium dilution, inert gas-wash out techniques, or any other conventional method known in the art.

Referring to FIGS. 5-6B together, as is demonstrated in FIGS. 6A and 6B, between steps 1 and 7, which are at a same airway pressure (i.e., PEEP), a baseline 604 amount of air remaining in the lung during end-expiration, i.e., the EELV, increased. The increased baseline amount of air is measured by a change in baseline impedance (($\Delta Z$), which may be measured or represented in milliliters of air depending on calibration procedures), despite the application of the same PEEP level before and after the steps 1 to 7. Furthermore, the increase baseline amount of air indicates that at least some alveoli were recruited between steps 1 and 7. Furthermore, as indicated in FIGS. 6A and 6B, amplitudes of the later pulses of inspiratory driving-pressures indicated in the EIT plethysmogram having the same PEEP levels as earlier pulses of inspiratory driving-pressure indicate that the compliance of the lungs (i.e., an ability of the lungs to stretch and expand) of the patient is higher at the later pulses of inspiratory driving-pressures. For example, the patient is receiving a higher tidal

8

volume at the later pulses of inspiratory driving-pressures, indicating that a larger number of alveolar units are recruited and expanding during inspiration in response to the inspiratory pressures.

In some embodiments, an EELZ may be determined and/or measured for a first PEEP of the sequence of PEEPs, and an EELZ may be determined and/or measured for a second PEEP of the sequence of PEEPs applied to the patient. Furthermore, an EELZ may be determined and/or measured for a third, fourth, fifth, sixth, seventh, eight or any number of subsequent PEEPs applied to the patient.

Referring to FIG. 5, the method 500 may further include determining (e.g., calculating) changes in EELZ between applied PEEPS of the sequence of applied PEEPs, as shown in act 515 of FIG. 5. For instance, act 515 may include determining a change in EELZ between a first applied PEEP and a second subsequently applied PEEP (e.g., calculating the difference between a first EELZ and a second EELI). In some embodiments, the change in EELZ may be determined from EIT images generated at each PEEP of the sequence of applied PEEPs (discussed in greater detail in regard to act 520 of FIG. 5). The change in EELZ may be representative of a change in EELV. For instance, in some embodiments, the method 500 may further include determining a change in EELV based on the change in EELZ.

In some instances, the first applied PEEP and the second applied PEEP may be immediately sequential to each other. For instance, the first applied PEEP may be applied in step 1, as depicted in FIGS. 6A and 6B, and the second applied PEEP may be applied in step 2, as depicted in FIGS. 6A and 6B. In other embodiments, the first applied PEEP and the second applied PEEP may be separated by at least one other application of a PEEP. In some embodiments, the first applied PEEP and the second applied PEEP may be at least substantially the same amount of applied pressure (e.g., steps 1 and 7 of FIGS. 6A and 6B). In such instances, the first applied PEEP and the second applied PEEP may have at least one application of an increase in PEEP between the first applied PEEP and the second applied PEEP.

In one or more non-claimed embodiments, the method 500 may further include determining a chord-compliance (or pressure-volume relationship) of the lungs (e.g., at least one region of a lung) of the patient at each applied PEEP of the applied sequence of PEEPs, as shown in act 520 of FIG. 5. In some embodiments, the act 520 may include obtaining an EIT image of at least a portion of the lungs of the patient at each applied PEEP of the sequence of applied PEEPs. For example, act 520 may include generating (e.g., reconstructing) EIT images via any of the manners described above in regard to FIGS. 1-3 and utilizing any of the EIT systems described above in regard to FIGS. 1-4.

In some embodiments, act 520 may include determining a chord-compliance of the lungs of the patient on a pixel by pixel level. For instance, act 520 may include determining a chord-compliance indicated by each pixel of each generated EIT image based at least partially on the impedance indicated by each pixel of each generated EIT image.

Referring to FIGS. 5-6B together, the EIT plethysmograph includes a waveform that is derived from a sum of all pixels within a given region of interest of an EIT image (or the entire EIT image) (frame) plotted against time. In particular, the EIT plethysmograph represents an amount of air that moves in and out of the region of interest (referred to as tidal oscillation ($\Delta Z_{VT}$)). The tidal oscillation ($\Delta Z_{VT}$) in the EIT plethysmograph correlates to a change in lung volume estimated by computerized tomography. Additionally, the determined change in EELVs (acts 510 and 515) correlates to the change in end-expiratory lung impedance (EELZ) demonstrated in the generated EIT images. Thus, the generated EIT images indicate changes in pulmonary aeration (via $\Delta$EELZ) caused by, for example, position changes or PEEP adjustments and/or changes.

In view of the foregoing, the EIT images are a representation of the tidal changes in impedance pixel by pixel. In other words, the EIT images represent a color map of the pixel wise $\Delta Z$ (e.g., $\Delta Z_{VT}$). Accordingly, based on the EIT images, a distribution of ventilation in given direction (e.g., ventral-to-dorsal direction) for a given PEEP can be determined. Furthermore, based on the pixel wise $\Delta Z$, at each PEEP step, a compliance can be calculated from an amount of air entering the lungs ($\Delta Z$) and the difference between a plateau pressure ($P_{plateau}$) and the applied PEEP (e.g., an elastic pressure of the lungs). For instance, because the plateau pressure ($P_{plateau}$) and the applied PEEP can be substituted for inspiratory and expiratory alveolar pressures at zero flow, a compliance of each EIT image pixel can be estimated as:

$$(1)\ Compliance_{pixel} = \frac{\Delta Z}{P_{plateau} - PEEP}$$

Based on the determined compliance of each pixel, a sum of the determined compliances of each pixel of a given EIT image yields an overall compliance of the image (e.g., an overall compliance of the lungs of the patients at a given PEEP). Likewise, a sum of the determined compliances of each pixel within a region of interest of an EIT image yields an overall compliance of the region of interest.

Moreover, the method 500 may include calculating a first index representing a change in EELZ in the lungs of the patient based on a chord compliance and/or an overall compliance determined in act 520, as shown in act 525 of FIG. 5. For instance, act 525 may include calculating an first index (e.g., a value and/or continuous variable) representing the change in EELZ in at least one region of a lung of the patient that is "above-predicted" or "below-predicted" based on a first chord-compliance (a pressure-volume relationship) observed during a first given applied PEEP. For example, the first index may be at least partially representative of how far above or how far below the change in EELZ is relative to a predicted change in EELZ for given alveolar pressure. The first index, the change in EELZ, and procedures for determining the change in EELZ is described in greater detail below in regard to, for example, FIGS. 7-9.

Additionally, the method 500 may include determining a change in chord-compliance and/or overall compliance (e.g., a change in lung compliance), as shown in act 530. For instance, act 530 may include determining a change in chord-compliance of the lungs of a patient and/or determining a change in chord-compliance of a region of interest of the lungs of the patient. For instance, act 530 may include determining a change in a chord-compliance (or a pixel compliance) between a first applied PEEP and a second subsequently applied PEEP (e.g., calculating the difference between a first determined chord-compliance and a second determined chord-compliance).

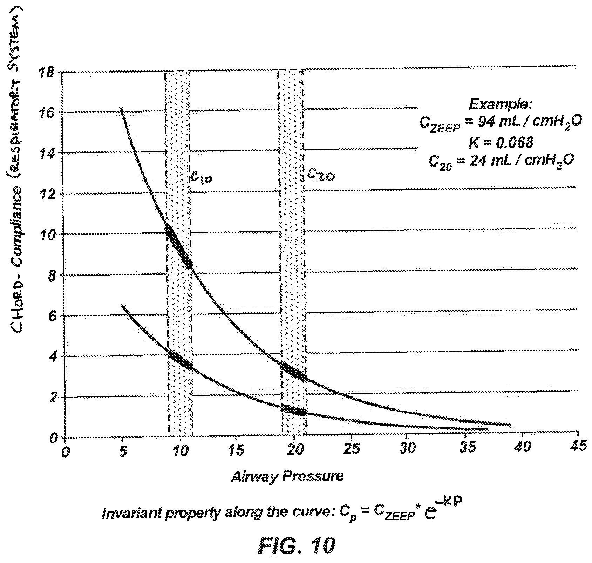

Furthermore, the method 500 may include calculating a second index (e.g., a value) representing the determined change in chord-compliance in the lungs of the patient (e.g., at least one region of a lung of the patient), as shown in act 530a of FIG. 5A. Additionally, act 530a may include calculating a second index that is "above-predicted" or "below-predicted" based on the pressure difference between the first applied PEEP and the second subsequently applied PEEP and based on algorithms describing the elastic lung behavior, such as any of the equations described below in regard to FIGS. 7-14. For example, the second index may be at least partially representative of how far below or how far above the change in compliance is relative to a predicted change in compliance for given alveolar pressure. The second index, the change in compliance, and procedures for determining the change in compliance is described in greater detail below in regard to, for example, FIG. 10.

In some instances, the first applied PEEP and the second applied PEEP may be immediately sequential to each other. For instance, the first applied PEEP may be applied in step 1, as depicted in FIGS. 6A and 6B, and the second applied PEEP may be applied in step 2, as depicted in FIGS. 6A and 6B. In other embodiments, the first applied PEEP and the second applied PEEP may be separated by at least one other application of a PEEP.

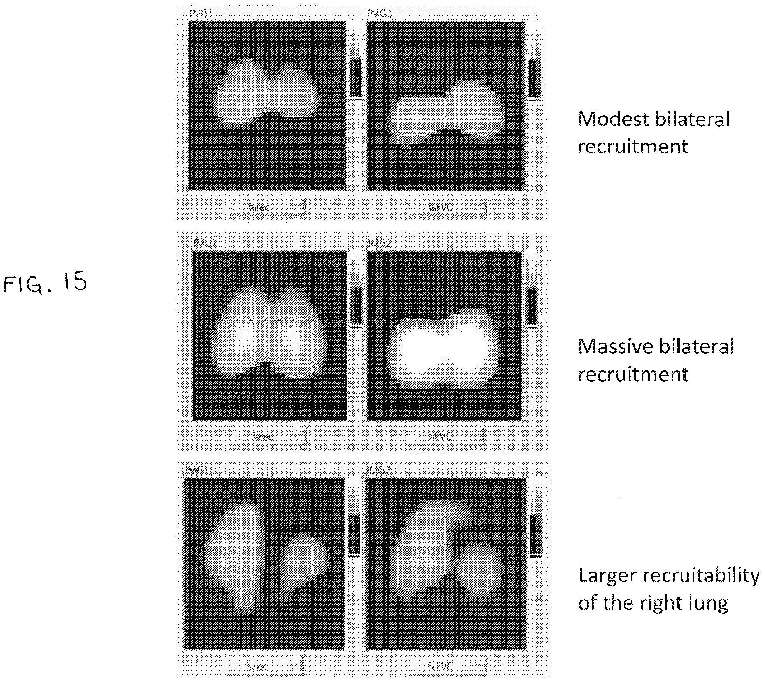

Furthermore, the method 500 may include determining an amount of alveoli (e.g., a volume of the lungs) recruited by applying the sequence of PEEPs (i.e., PEEP therapy), as shown in act 535 of FIG. 5. Furthermore, as will be discussed in greater detail in regard to FIGS. 7-15, act 535 of FIG. 5 may include normalizing the first and second indexes (e.g., the values and/or continuous variables constituting the first and second indexes) representing the determined change in EELZ and the determined change in compliance, as shown in act 540 of FIG. 5 and applying weights to (e.g., weighting) the first and second indexes representing the determined change in EELZ and the determined change in compliance and measurements of compliance and EELI, as shown in act 545 of FIG. 5. Applying the weights to the first and second indexes is described in greater detail below in regard to FIG. 15. In some embodiments, acts 540 and 545 of FIG. 5 may be independent of act 535. Furthermore, in some embodiments, act 535 may be optional and may not be required in every embodiment.

FIG. 7 shows a graph of the lung volume of the patient (y-axis) determined from an EIT plethysmograph (e.g., the plethysmograph of FIG. 6B) plotted against a determined (e.g., calculated) alveolar pressure (x-axis). Referring to FIGS. 5 and 7 together, each segmented line connects (e.g., extends between) points representing a moment of end-expiration and a moment of end-inspiration at each step of applied PEEP and represents a chord compliance ($\Delta V / \Delta P$) of the lungs of the patient that links an end-expiratory pressure-volume to end inspiratory pressure-volume at a given applied PEEP. The sequence of applied PEEPs in the example of FIG. 7 corresponds to FIG. 6B. Continuous line 701 (corresponding to step 0) represents a moment of PEEP=10 cmH$_2$O with inspiratory alveolar pressures of 20 cmH$_2$O. Dashed line 702 represents an extrapolation of the continuous line 701 to higher pressures. The dashed line 702 may be extrapolated via, for example, linear extrapolation, exponential extrapolation with downward concavity, etc. Dashed line 702 represents predicted lung volumes for a given alveolar pressure. Arrow 703 represents an EELV (and as a result an EELZ) gain "above-predicted" within the lung caused by alveolar recruitment. Furthermore, a vertical distance (indicated by arrow 703) between steps of the same applied PEEP (e.g., steps 1 and 7, which have the same PEEP of 15 cmH$_2$0) is proportional to a recruited volume (volume-gain) of the lungs at this respective pressure. In some embodiments, additional plots and/or curves such as those depicted in FIG. 7 may be plotted for at least one region of interest of a lung (e.g., for just one region of interest of a lung) of the patient or individually for each pixel of an EIT image generated for a given applied PEEP, where the y-axis is a volume change in the region of interest or pixel. In FIG. 7, the origin of the y-axis is zero, representing a functional residual capacity for a given individual at a first PEEP step.

In some embodiments, the volume of the patient's lungs may be determined by converting the captured EIT signals of the EIT images into volume in millimeters via conventional calibration methods.

However, referring still to FIGS. 5 and 7 together, unlike conventional methods of determining a recruited volume of lungs of a patient, the system and methods described herein account for the occurrence that a similar number of alveoli may be represented by different vertical distance (e.g., differences in volume) depending on the amount of applied PEEP. For instance, a same number of recruited alveoli may be indicated by a relatively larger vertical distance (e.g., change in volume between applied PEEPs) at a relatively higher applied PEEP and by a relatively smaller vertical distance (e.g., change in volume between applied PEEPs) at a relatively lower applied PEEP. For instance, referring to FIG. 8, which shows a graph of a number of recruited alveoli at a first PEEP$_A$ and at a second PEEP$_B$, because PEEP$_B$ is greater than

$PEEP_A$, the vertical distance (e.g., the volume-gain between applied PEEPs) is larger at PEEPs. In particular, FIG. 8 shows exponential pressure-volume curves representing simulated lung inflation when all lung alveoli (e.g., units) are already recruited at a start of inflation. For the sake of simplicity, the represented lung has only includes two units. The dashed line 801 represents a case where one lung unit was recruited after the beginning of lung inflation, and the solid line 802 represents the case where two lung units were recruited after the beginning of lung inflation. The shadowed zones 805 represent tidal-oscillations in alveolar pressures during the first step with $PEEP_A$ (around 10 $cmH_2O$) and the second step with $PEEP_B$ (around 20 $cmH_2O$).

To calculate the chord compliance at a certain PEEP step, the slope of the portions of the exponential functions within the shadowed zones is measured. The arrows 803 represent the volume-gain "above-predicted" for a patient starting inflation with one unit recruited, and subsequently having another unit recruited. When no recruitment occurs, the "predicted-volume" is the volume predicted by the exponential curve at a higher pressure (next PEEP step), which can be approximated by a line with same slope of the chord compliance when the PEEP steps are close enough. As shown in FIG. 8, volume gain is larger at higher pressure following a relationship that can be calculated (and accounted for) according to one of the embodiments of the present disclosure. In particular, the method 500 may include adjusting the values of PEEP by, for example, fitting the applied PEEPs to a linear function or exponential function. For instance, the method 500 may include fitting the applied PEEPs to the following function:

$$(2)\ \%Recruitment = \frac{Volume_{gain}\,at\,PEEP_B}{FVC_{predicted}*(1-e^{-K.B})}$$

where $Volume_{gain}$ represents the volume gain observed at PEEP = B, (e.g., between steps 1 and 7), $FVC_{(predicted)}$ represents the predicted force vital capacity obtained via conventional formulas and methods of pulmonary function tests after accounting for the patient's individual anthropometric measurements (e.g., sex, height, weight, and race), and K is the constant described in regard to FIGS. 8-10 herein and defining the specific hyper-elastic behavior of the lung and chest-wall during inflation. Value K can be fixed (e.g. 0.06), or can be measured for the same individual, during decremental PEEP steps.

Additionally, method 500 (e.g., act 535) of FIG. 5 may include converting a determined recruited volume of the lungs of the patient into a percentage of recruited lung mass and/or units. In particular, the method 500 may include determining changes in chord-compliance between steps applying the same PEEP by estimating a weighted sum of $C_P$ (pixel compliance) for each step of applying PEEP=B. In estimating the weighted sum of $C_P$, it is assumed that $C_{ZEEP}$ (chord compliance at ZEEP) is a function of a size of the patient, which can be related to the predicted forced vital capacity according to the formulas below:

$$(3)\ C_B = C_{ZEEP} * e^{-KB}$$

$$(4)\ FVC_{predicted} = \left(\frac{C_{ZEEP}}{K}\right)$$

where $C_B$ represents the chord-compliance (of a respiratory system) measured at PEEP = B and using a relatively small tidal volume (e.g., smallest possible tidal volume), $C_{ZEEP}$ represents the chord-compliance (of the respiratory system) measured at zero end expiratory airway pressure (ZEEP) and using the relatively small tidal volume (e.g., smallest possible tidal volume), and K is the constant described herein in regard to FIGS. 8-10 herein, defining the specific hyper-elastic behavior of the lung and chest-wall during inflation. Value K can be fixed (e.g. 0.06), or can be measured for the same individual, during decremental PEEP steps.

Moreover, by measuring the chord-compliance ($C_A$) at any PEEP level = A, the expected chord-compliance $C_B$ at PEEP = B is predictable by using the formula shown below, which is derived from equations 3 and/or 4 above:

$$(5)\ C_A = C_B * e^{-K(B-A)}$$

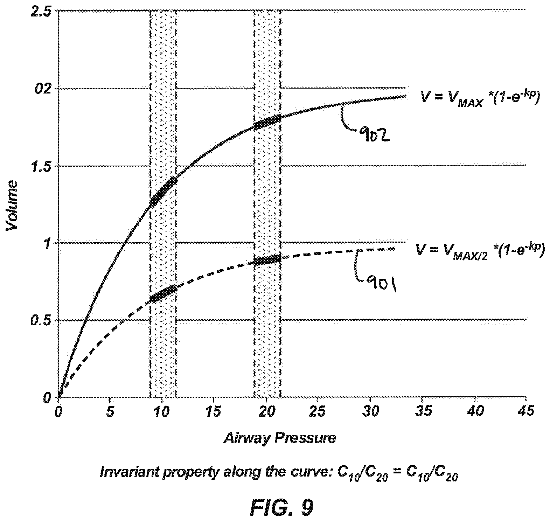

FIG. 9 shows a graph of simulated lung inflation including volume of lungs plotted against applied PEEPs. FIG. 9 further includes equations representing dashed inflation line 901 and solid inflation line 902, where V represents the lung volume as a certain alveolar pressure p, $V_{max}$ represents an asymptotic maximum lung volume at an infinite alveolar pressure, and value K is the constant defining the hyper-elastic behavior of the lung and chest-wall during inflation. Unlike a linear model of inflation, the elasticity of the lung and chest decreases at higher pressure according to the shown exponential functions and the value, K. The shown exponential functions are validated in physiological studies of humans as shown in Knudson RJ, Kaltenborn WT. Evaluation of lung elastic recoil by exponential curve analysis. Respir Physiol.

1981 Oct; 46(1):29-42. However, the exponential functions of FIG. 9 are adjusted to represent the behavior of the lung plus chest-wall together. Additionally, the lung volume at the origin of the graph is assumed to be the functional residual capacity (FRC) at zero-PEEP.

The methods described above in regard to FIGS. 5-9 provide advantages over conventional methods because $V_{MAX}$ can be approximated to total lung capacity ("TLC") or to vital Capacity ("FVC") when the origin of the graph is assumed to be the residual volume of the lung. As is known in the art, the FVC can be predicted via conventional equations extracted from human populations and using anthropometric information from the patient, such as, age, height, sex, weight, and/or race. Thus, by knowing the predicted FVC for a patient, the FVC can be compared to the observed-volume-gain "above-predicted" during a sequence of PEEP steps. After extrapolating the observed-volume-gain to an infinite alveolar pressure (using the same exponential functions), the method 500 may include calculating the ratio between this asymptotic-volume-gain and the asymptotic $V_{MAX}$, which represents the percentage of lung units that were recruited from $PEEP_A$ to PEEPs.

FIG. 10 shows a graph of calculated lung compliances plotted against applied PEEPs utilizing the same simulated lung model with only two units, as represented and described in regard to FIGS. 8 and 9. In FIG. 10, the chord compliance represents a derivative of the exponential functions describing lung and chest-wall inflation discuss above in regard to FIGS. 8 and 9. In view of the foregoing, the compliance at a certain pressure (PEEP) can be calculated according to the following equation:

$$(6)\ C_B = C_{ZEEP} * e^{-KB}$$

where $C_B$ represents the chord compliance (of respiratory system) measured at PEEP = B and using a relatively small tidal volume (e.g., smallest possible tidal volume), $C_{ZEEP}$ represents the chord compliance (of the respiratory system) measured at zero end expiratory airway pressure (ZEEP) and using the relatively small tidal volume (e.g., smallest possible tidal volume), and K is the constant described herein in regard to FIGS. 8-10, defining the specific hyper-elastic behavior of the lung and chest-wall during inflation. Value K can be fixed (e.g. 0.06), or can be measured for the same individual, during decremental PEEP steps.

The methods described in regard to FIG. 10 are advantageous over conventional methods because, after the constant K is determined, and the compliance $C_{ZEEP}$ measured at PEEP = 0, the expected compliance at any other pressure can be predicted which enables a determination as to whether an observed change in compliance is "above-predicted" (i.e., above the predicted compliance) or "below-predicted" (i.e., below the predicted compliance). Additionally, a decrease in chord compliance should be expected from an increase in PEEP. However, if the determined change in compliance is "above-predicted" (i.e., the decrease is less than expected), the change in compliance indicates lung recruitment. Conversely, if the determined change in compliance is "below-predicted," the change in compliance indicates derecruitment.

Moreover, by measuring the chord-compliance ($C_A$) at any PEEP level = A, the expected chord compliance $C_B$ at PEEP = B can be predicted using the formula below, which is derived from the equation above.

$$(7)\ C_A = C_B * e^{-K(B-A)}$$

Moreover, in view of the exponential relationships described above, the predicted FVC is related to a predicted chord-compliance at ZEEP, according to the following equation:

$$(8)\ FVC_{predicted} = \frac{C_{ZEEP}}{K}$$

where $FVC_{predicted}$ is the predicted forced-vital-capacity, $C_{ZEEP}$ is the predicted chord-compliance when PEEP = 0, and K is the constant described herein in regard to FIGS. 8-10, defining the specific hyper-elastic behavior of the lung and chest-wall during inflation. Value K can be fixed (e.g. 0.06), or can be measured for the same individual, during decremental PEEP steps.

Utilizing the equations described above, the compliance (e.g., observed compliance) can be measured at a certain PEEP, predicted at PEEP = 0, and related to the predicted compliance at zero PEEP (CZEEP) utilizing conventional equations related to FVC.

Referring still to FIGS. 5, 9, and 10 together, factor K can be extracted by assuming that typical aerated parenchyma (relatively large number of alveoli) obey an exponential inflation according to the equations indicated in FIG. 9. Furthermore, it is assumed that along the curves represented by the equations indicated in FIG. 9, given a first PEEP (e.g., 10 cmH$_2$O) along both curves and a second PEEP (e.g., 20 cmH$_2$O) along both curves, a compliance of a first curve at the first PEEP divided by a compliance of the first curve at the second PEEP is always equal to a compliance of a second curve at the first PEEP divided by a compliance of the second curve at the second PEEP (e.g., $C_{10}/C_{20}$ of the first curve is equal to $C_{10}/C_{20}$ of the second curve).

Based on the foregoing, lung compliance of normally aerated parenchyma can be demonstrated to obey an exponential decay along increasing PEEP levels according to the equations indicated in FIG. 10. Furthermore, $C_P = C_{ZEEP}*e^{-kP}$ is shown to be an invariant property in FIG. 10. Additionally, factor K is extracted by assuming that, once open, the parenchyma obeys the equations indicated in FIGS. 9 and 10. Furthermore, collapsed lung tissue represents some lung units that are excluded from the exponential inflation. The collapsed lung tissue remains closed, causing a decrease in potential $V_{MAX}$ (FIG. 9). For example, if half of the units of a lung are closed at a PEEP of 40cmH$_2$O, the lung inflation can be predicted as $V = V_{MAX}/2 * (1-e^{-kp})$.

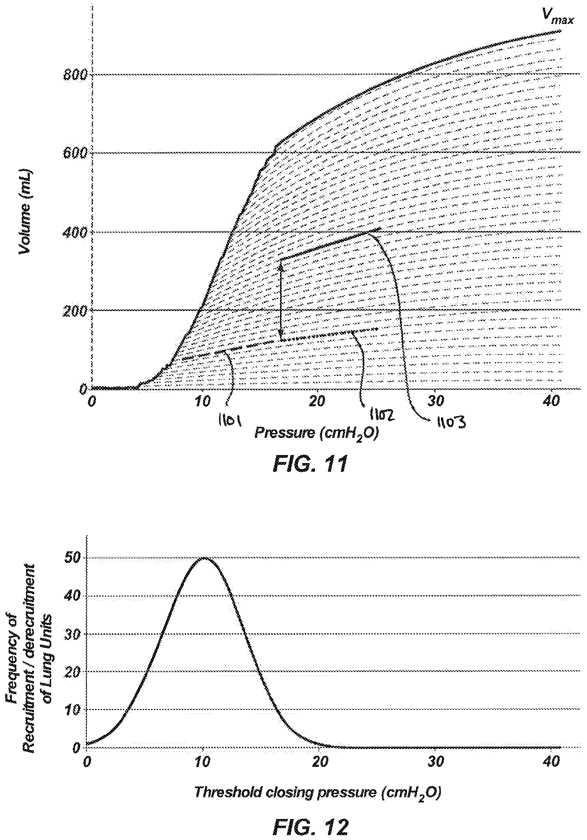

FIG. 11 shows simulated pressure-volume relationships during lung inflation while modeling a lung with multiple compartments in which each compartment behaves like simpler models (e.g., the models described above in regard to FIGS. 8-10). However, the simulated pressure-volume relationships demonstrated in FIG. 11 are based on the units (e.g., all units) being closed at the origin when airway pressures are zero. As shown in FIG. 11, even if each compartment obeys an exponential inflation (once recruited), the sequential recruitment of lung units at different airway pressures produces a sigmoidal pressure-volume relationship for the whole lung inflation (black thick line), because the recruitment of each unit produces sequential and small "volume-gains" as indicate in by the arrow 803 of FIG. 8. Thus, even for patients with lungs typically presenting sigmoidal pressure-volume relationships, the models and equations described herein to calculate the volume gain remain accurate. When performing the maneuvers described in the present disclosure, the chord-compliance during PEEP$_A$ can be represented by the slope of the segment 1101 and the chord compliance during PEEPs is represented by the slope of the segment 1102. Additionally, the slope of the segment 1102 represents the predicted-compliance at PEEPs if no recruitment has occurred. As shown, the higher slope of the segment 1103 in comparison to the theoretical slope of the segment 1102 (the predicted one) indicates that there was relatively large amounts of recruitment of new lung units.

FIG. 12 shows a function defining a probability or frequency of occurrence of derecruitment of the lungs of a patient. For this example, the threshold opening pressures are set and simplified as 40 cmH$_2$O for the entire lung. For instance, there is no tidal recruitment if plateau pressures do not reach 40 cmH$_2$O. The foregoing simplification remains accurate when applying to low tidal volumes. In particular, FIG. 12 represents, mathematically, the derivative of the sigmoidal curves shown in FIG. 11.

The sigmoidal curves of FIG. 11 represent a composition of exponential curves, in which a lung size is progressively larger (i.e., progressively moving (e.g., jumping) to an upper exponential curve) when an applied PEEP is above a threshold closing pressure of parenchyma. Furthermore, the sigmoid curves of FIG. 11 assume that a currently applied PEEP is applied after a recruitment maneuver occurred that exceeded 40 cmH$_2$O. A low probability of derecruitment at very low (around zero) or at very high pressures (around 20 cmH$_2$O with the peak probability occurring at 10 cmH$_2$O) cause the sigmoid shape of the derecruitment curve with a maximum slope at 10 cmH$_2$O. The maximum slope at 10 cmH$_2$O indicates a relatively large derecruitment is expected around this 10 cmH$_2$O).

Referring to FIGS. 5-12 together, the foregoing demonstrates that if an applied PEEP is kept at a same value before and after a recruiting maneuver, a percent increase in compliance (i.e., a percentage of the predicted value) is proportional to a gain in the percent mass of aerated-lung tissue (e.g., recruited amount of lung). Furthermore, the foregoing shows that a percent-mass of the ventilated lung is proportional to the percent-number of ventilated lung units, and that the percent-number of ventilated lung units is proportional to the compliance of respiratory system.

In some embodiments, a percentage of the mass or a number of units recruited by applying the sequence of PEEPs to the lungs of the patient is determined the equations described above in regard to FIGS. 5-12. Furthermore, in some embodiments, the first and second indexes may be at least partially determined via the equations described above in regard to FIGS. 5-12. For instance, the above-described calculations, which are based on the change in end expiratory lung volume and the change in chord-compliance (e.g., the first and second indexes), may indicate that a certain percentage of the lungs was recruited during the application of the sequence of PEEPs. For example, the recruited percentage may be between 0 and 5%, 5% and 10%, 10% and 20%, or over 20%.

The recruited percentage may indicate whether that the collapsed parenchyma of the lungs exhibit a relatively low threshold opening pressure. For instance, the recruited percentage may indicate that the collapsed parenchyma of the lungs exhibit a threshold opening pressure of less than 50 cmH$_2$O. Having a threshold opening pressure of less than 50 cmH$_2$O suggests that the cost (e.g., risk of hemodynamic impairment and barotrauma) of a recruitment maneuver is relatively low when compared to the benefits (e.g., relatively large reduction in driving pressures, pulmonary shunt, and dead space) after a recruiting maneuver.

As mentioned above, in some embodiments, act 535 may include applying weights to the values (e.g., the first and second indexes) representing the determined change in EELZ and the determined change in compliance and measurements of compliance and EELI, as shown in act 545 of FIG. 5. Applying the weights to the first and second indexes is described in greater detail below in regard to FIG. 15.

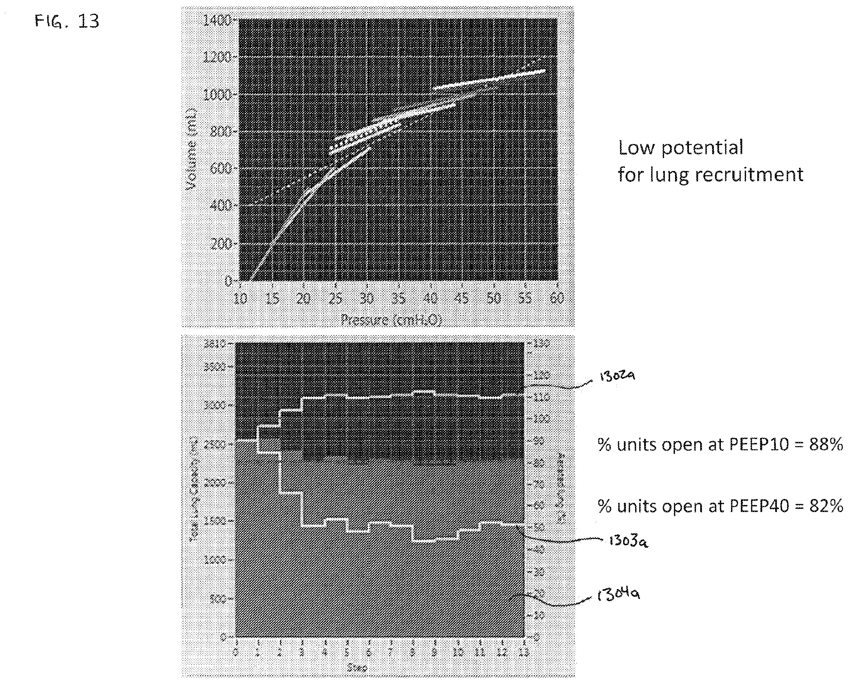

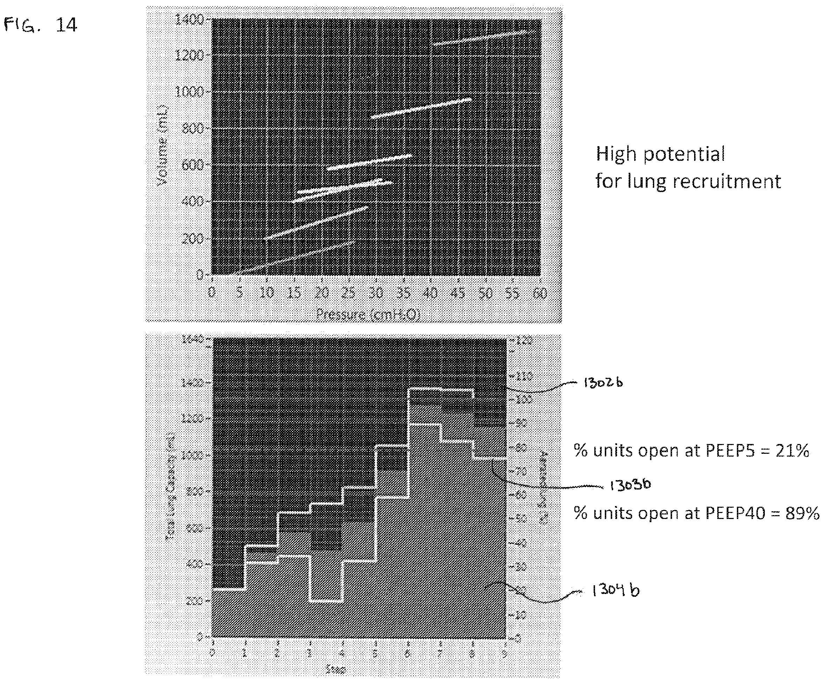

The method 500 may further include, based on the normalized and/or weighted first and second indexes, determining a potential lung recruitment value for the patient, as show in act 550 of FIG. 5. Furthermore, act 550 may include determining a threshold value and classifying the patient as a responder (e.g., an individual that would respond well to a recruitment maneuver) of a non-responder (e.g., an individual that would not respond well to a recruitment maneuver) based on the threshold value. FIGS. 13 and 14 are referred to in conjunction with FIG. 5 to describe the procedures and methods of determining the potential lung recruitment value. FIGS. 13 and 14 show graphs representing two different individuals, the first with a normal lung and PEEP = 10 cmH$_2$O and with very small recruitable lung tissue (i.e., no lung collapse when starting the maneuver), compared with the second who had a lung injury and has a relatively large potential for recruitment. The graphs were determined utilizing at least some of the methods described above in regard to FIG. 7.

In the upper panels of FIGS. 13 and 14, the pressure-volume relationships during the PEEP step maneuvers are represented where the lung volume is calculated from a generated EIT plethysmogram (e.g., the EIT plethysmograms described above in regard to FIGS. 6A and 6B). The volumes are plotted against the calculated alveolar pressure. Each line connects (e.g., extends between) points representing a moment of end-expiration and a moment of end-inspiration at each step of applied PEEP and represents a chord compliance ($\Delta V$ / $\Delta P$) of the lungs of the patient that links an end-expiratory pressure-volume to end inspiratory pressure-volume at a given applied PEEP. The sequence of PEEP steps is represented by different segments, ranging from PEEP$_A$=12cmH$_2$O to PEEP$_B$=40cmH$_2$O. The slope of each segment represents the chord compliance at each PEEP step. The segments present minimum vertical displacement in FIG. 13 (i.e., no volume gain "above-predicted," according to an exponential lung inflation), but the segments present a large vertical displacement in FIG. 14.

Additionally, the slopes of the segments markedly decrease along increasing pressures in FIG. 13, whereas the slopes are similar to each other in FIG. 14. The foregoing indicates that the slopes decreased according to an exponential decay in the first case, whereas the slopes remained "above-predicted" at higher pressures in the second case. Thus, the first and second indexes of lung recruitment were coherent, which indicates a non-responder in FIG. 13 and a responder in FIG. 14. The lower panels in each of FIGS. 13 and 14 show the evolution of the indexes for lung recruitment according to one or more embodiments of the present disclosure along the PEEP steps (shown in the x-axis). The horizontal solid lines represent the predicted FVC according to the anthropometric data from the patient assigned as 100%.

Referring still to FIGS. 13 and 14, at each new PEEP level, the volume-gain "above-predicted" (e.g., the first index) is calculated (upper stepped line 1302a, 1302b), and, after a sequence PEEPs, if this volume-gain approaches the FVC, the index reaches 100%. Simultaneously, the chord compliance (observed) (e.g., the second index) (lower stepped line 1303a, 1303b) is determined at each PEEP utilizing data obtained via EIT, and then is related to the "predicted-compliance" at ZEEP, which is derived from the predicted FVC according to the embodiments described herein. When the index reaches 100%, it indicates that the lung, at the given PEEP step, is demonstrating a same chord compliance that should be observed in a patient with normal lungs with the same anthropometric measurements and subjected to the same PEEP level. Additionally, the solid lower stepped portions 1304a, 1304b represent the weighted sum of the two indexes (e.g., the first index and the second index) according to one or more embodiments of the present disclosure. The percentages found at the first and last step of the maneuver are shown in numbers on the right. The weighted sum is utilized to minimize measurement errors that may occur during the computation of the two (or more) indexes.

Referring to FIGS. 5-14 together, the determined recruited percentage of the lungs of the patient indicates a level of physiologic dose-response behavior (represented in FIG. 13) that can be expected in an individual. In particular, for an individual having a relatively good potential for lung recruitment, at the lower levels of PEEPs (i.e., pressures lower than pressures utilized to achieve maximum lung recruitment), a measureable amount of the lung is recruited by applying the sequence of PEEPs. Alternatively, for an individual having a relatively poor potential for lung recruitment, at the lower levels of PEEPs, a little to no measureable amount of the lung is recruited by applying the sequence of PEEPs.

Because the determined indexes (e.g., the first index and the second index) can be continuous variables, and due to the determined indices weighted sums and/or averages (described in greater detail below in regard to FIG. 15), a threshold for a binary classification, 1) responder and 2) non-responder, may include at least the following embodiments.

Some embodiments include determining a threshold value based on a receiver-operating-characteristic-curve ("ROC") where multiple levels for the threshold value are tested using a binary outcome and a consequent performance of threshold based on its sensitivity and specificity. For example, a positive outcome (indicating a responder) may be a) an increase in oxygenation (expressed by a P/F ratio, or partial pressure of oxygen measured in the blood, divided by the oxygen faction used in the inspiratory gases) of more than 100, b) patient survival, c) bilateral improvement in radiography of the thorax, or d) an improvement in lung compliance of more than 25%. In some embodiments, the threshold value may include an increase of at least 20% (percentage points) in a combined index (in relation to the maximum predicted for a patient based on his/her anthropometric characteristics).

One or more embodiments include determining a threshold value based on initial data of patient after calculating the amount of recruitment needed to reduce the patient's inspiratory driving pressure by more than 5 cmH$_2$O. For instance, the threshold value could be based in a probable reduction in driving pressure of 3 cmH$_2$O. For example, the threshold value may be determined via the following equation:

$$(9) \ Threshold_{recruit \ index} = \frac{\Delta\Delta P * C_B^2}{V_T * C_{PRED} - \Delta\Delta P * C_{PRED} * C_B}$$

where $\Delta\Delta P$ is the intended reduction inspiratory driving pressures (e.g., 5 cmH$_2$O), $C_B$ is the compliance observed at the start of the RAM procedure (step 0), $V_T$ is the tidal volume to be used after the recruitment (e.g., 6 mL/kg), and $C_{PRED}$ is the predicted compliance for a normal lung subjected to PEEP used at baseline (or after recruitment) obtained via the methods described above, after determining the FVC from anthropometric data and published formulas for human populations. As a non-limiting example, for a common ARDS patient with observed $C_B$ = 20 mL/cmH$_2$O, $V_T$ = 400ml, baseline PEEP = 10 cmH$_2$O, $C_{PRED}$ = 54 mL/cmH$_2$O, and a target reduction in driving pressure of 5 cmH$_2$O, the threshold value (e.g., threshold value for a recruitability index) separating responders from non-responders would be 12%. The foregoing indicates that, if during a RAM, and increase of greater than 12% is observed in the combined indexes (recruitability index (i.e., the weighted sum of the first and second index)), the patient would be considered a responder having a relatively good potential to reduce the patient's driving pressure by 5 cmH$_2$O.

In some embodiments, act 550 may include determining a potential recruitment value of a region of interest of the lungs (e.g., only a portion of the lungs) of the patient that may be effectively recruited during an ARM. For instance, utilizing the EIT system and compliance values derivable therefrom, the potential recruitment value may be determined for a region of interest (e.g., only a portion of the lungs). In some embodiments, the region of interest may be in the axial plane of the patient or the sagittal plane of the patient.

FIG. 15 shows EIT images of three patients collected simultaneously where the first index is represented in the left column of images and the second index is represented in the right column of images. Utilizing more than one index in determining lung recruitment value of a patient is advantageous. For instance, utilizing more than one index decreases experimental errors assuming that the indexes originate from independent measurements. As a result, any errors are diluted due to averaging processes among various indexes. Additionally, there is a possibility that one index will better represent a recruitability of some or all of alveolar units (e.g., dependent units located in dorsal lung regions) and another index will better represent a recruitability of other alveolar units (e.g., independent units located in ventral lung regions). As demonstrated in the present disclosure, a first index may related to a volume gain of pop-open alveolar units (i.e., vertical displacement in the pressure volume plots described above) and may be more sensitive to recruitment within an anterior lung zones, and a second index may be related to pixel-chord compliance and pixel-chord compliance changes along multiple PEEP steps and may be more sensitive to recruitment in a posterior lung zones.

Referring still to FIG. 15, regions presented in lighter shades (i.e., larger recruitment) are displaced upward as compared to the right columns. The images in FIG. 15 demonstrate that the second index better represents recruitment of the dorsal zones of the lungs. As a result, the composition (e.g., combination) of the first index and the second index provides a more accurate (e.g., sensitive) detection of recruitment in comparison to conventional methods.

As noted above in regard to FIG. 5, in some embodiments, act 550 may include assigning a relative weight to each of the first index and the second index. In some embodiments, the relative weights may be fixed across a certain patient population, or estimated case by case. As mentioned above, the relative weights help to correct for a well-known phenomenon that, at high PEEP levels, the gain in lung volume is disproportionally high as compared to the gain in compliance.

In one or more embodiments, when the first index and the second index are utilized in conjunction with each other to dilute errors, each of the first index and the second index may be assigned balanced weights (e.g., 50% each). In additional embodiments, the weights of the first and second indexes may be assigned according to the reliability of the measurements utilized in determining the first and second indexes. For instance, the weights of the first and second indexes could be inverse to coefficients of variation for the respective indexes. In further embodiments, the weights of the first and second indexes may be assigned as a progressive balance across the pixels of the EIT images where the pixels located in the dorsal regions have a higher weight for the second index (e.g., 75%) and where the pixels in the ventral regions have a higher weight for the first index (e.g., 75%). In view of the foregoing, the weights of the first and second indexes may be assigned for each pixel based on a spatial location of the pixel.

Referring to FIGS. 1-15 together, the systems and methods described herein may provide advantages over conventional systems and method for determining recruitment potential of lungs of a patient. For instance, due to at least the data provided by the EIT images, the systems and methods of the present disclosure may more accurately differentiate between stretching of an already-aerated lung and actual recruitment of previously collapsed when determining a recruited percentage of lungs of a patient when applying a sequence of PEEPs. As a result, the systems and methods of the present disclosure may provide a more accurate quantification of a potential lung recruitment value in comparison to conventional systems and methods. Additionally, unlike conventional systems and methods, the systems and methods of the present disclosure may be implements via EIT, by pulmonary mechanics utilizing gas monitoring sensors, or by any combination thereof. Furthermore, the systems and methods of the present disclosure may be implemented during tidal breaths within any additional required maneuvers beyond an application of a sequence of different PEEP levels.

Furthermore, unlike conventional systems and methods, the systems and methods of the present disclosure utilizes information at the pixel level of EIT images to assist in determining a potential lung recruitment value. In particular, systems and methods of the present disclosure utilizes pixel locations (e.g., locations within the lungs indicated by the pixels), changes in pixel aeration between images, and changes in pixel compliance during the application of the sequence of different PEEP levels. As a result, the systems and methods of the present disclosure can provide a more accurate quantification of a potential lung recruitment value. In particular, the systems and methods of the present disclosure may provide a spatial location within an EIT-cross-sectional image or EIT-3D image that is representative of a location within the lungs of the patient where recruitment is achievable. Additionally, the systems and methods of the present disclosure may estimate at a pixel level, a potential for recruitment.

Moreover, because the systems and methods of the present disclosure utilize EIT, at least portions of the systems and methods may be implemented bedside, used continuously, implemented in real time, and non-invasive. Furthermore, unlike computerized tomography, EIT does not require the use of radiation. Additionally, unlike pulmonary mechanics, EIT provides regional information and estimations of changes in EELV.

One or more embodiments of the present disclosure may include a method for determining a potential lung recruitment value for a patient, using an Electrical Impedance Tomography (EIT) system. The method may include applying a first positive end expiratory pressure to the lung of the patient; measuring a first end expiratory lung impedance in at least one region of the lung, that represents a first end expiratory lung volume at said region of the lung of the patient, at the first positive end expiratory pressure; applying a second positive end expiratory pressure to the lung of the patient; measuring a second end expiratory lung impedance in the at least one region of the lung, that represents a second end expiratory lung volume at the said region of the lung of the patient, at the second positive end expiratory pressure; determining a change in end expiratory lung impedance in the at least one region of the lung, that represents the change in end expiratory lung volume in said region of the lung, between the first positive end expiratory pressure and the second positive end expiratory pressure; determining a first chord-compliance (or pressure-volume relationships) of the at least one region of the lung of the patient from the corresponding region of EIT images of the patient, acquired at the first positive end expiratory pressure; calculating an index representing the change in end-expiratory lung impedance in the at least one region of the lung that is "above-predicted" or "below-predicted" based on the first chord-compliance (pressure-volume relationships) observed during the first positive end expiratory pressure; determining a second chord-compliance (or pressure-volume relationships) of the at least one region of the lung of the patient from the corresponding region of EIT images of the patient, acquired during tidal breaths at the second positive end expiratory pressure; calculating an index representing the change in lung compliance in the at least one region of the lung that is "above-predicted" or "below-predicted" based on the pressure difference between at least two positive end expiratory pressures and based on equations describing the elastic lung behavior; normalizing the indexes by predicted lung volumes like vital capacity, total lung capacity, residual capacity, or inspiratory capacity obtained from human population studies, and/or from anthropometric measurements of the patient; weighting the indexes and measurements of compliance and end expiratory lung impedance to determine the potential for lung recruitment of that at least one region of the lung of the patient; and classifying the patient as responder or non-responder.

While the present invention has been described herein with respect to certain illustrated embodiments, those of ordinary skill in the art will recognize and appreciate that it is not so limited. Rather, many additions, deletions, and modifications to the illustrated embodiments may be made without departing from the scope of the invention as hereinafter claimed. In addition, features from one embodiment may be combined with features of another embodiment while still being encompassed within the scope of the disclosure. Further, embodiments of the disclosure have utility with different and various detector types and configurations.

**Claims**

1.  A system for determining a potential lung recruitment value for a patient, the system comprising:

    an electrical impedance tomography device (404);
    at least one processor (428); and
    at least one non-transitory computer readable storage medium (430) storing instructions thereon that, when executed by the at least one processor, cause the at least one processor to:

    - in response to a sequence of positive end expiratory pressures being applied to a patient, cause an end expiratory lung impedance to be measured by the electrical impedance tomography device at each positive end expiratory pressure of the sequence of positive end expiratory pressures;
    - determine a first index representing a change in end expiratory lung impedance between a first end expiratory lung volume of the sequence of positive end expiratory pressures and a second, subsequent end expiratory lung volume of the sequence of positive end expiratory pressures;

- determine a second index representing a change in chord-compliance of the lungs of the patient between the first end expiratory lung volume and the second, subsequent end expiratory lung volume; and

- based on the first index and the second index, determine a potential lung recruitment value for the patient.

2. The system of claim 1, wherein determining a potential lung recruitment value for the patient comprises assigning a relative weight to each of the first index and the second index.

3. The system of claim 2, further comprising instructions that, when executed by the at least one processor, cause the at least one processor to assign the relative weights to the first index and the second index based on at least one of age, body mass index, or gender.

4. The system of claim 1, further comprising instructions that, when executed by the at least one processor, cause the at least one processor to determine a change in chord-compliance of the lungs, comprising:
determining a compliance represented by each pixel of a first electrical impedance

tomography image representing the first end expiratory lung volume; and determining a compliance represented by each pixel of a second electrical impedance
tomography image representing the second, subsequent end expiratory lung volume.

5. The system of claim 4, wherein determining a compliance represented by each pixel is determined by dividing an amount of air entering the at least one lung during a respiratory cycle by a difference between an inspiratory pressure at zero flow and a positive end expiratory pressure.

6. The system of claim 1, further comprising instructions that, when executed by the at least one processor, cause the at least one processor to classify the patient as either a responder or a non-responder based on whether the determined potential lung recruitment value for the patient meets or exceeds a threshold value.

7. The system of claim 4, further comprising a ventilation system and an electrical impedance tomography system operably coupled to the at least one processor.

**Patentansprüche**

1. System zum Bestimmen eines potentiellen Lungenrekrutierungswerts für einen Patienten, das System umfassend:

eine Vorrichtung (404) für eine elektrischen Impedanztomographie;
mindestens einen Prozessor (428); und
mindestens ein nicht flüchtiges computerlesbares Speichermedium (430), das Anweisungen darauf gespeichert aufweist, die, wenn sie durch den mindestens einen Prozessor ausgeführt werden, den mindestens einen Prozessor veranlassen zum:

- als Reaktion auf eine Abfolge von positiven endexspiratorischen Drücken, die auf einen Patienten angewendet werden, Veranlassen einer endexspiratorischen Lungenimpedanz, durch die Vorrichtung für die elektrische Impedanztomographie bei jedem positiven endexspiratorischen Druck der Abfolge von positiven endexspiratorischen Drücken gemessen zu werden,
- Bestimmen eines ersten Index, der eine Änderung der endexspiratorischen Lungenimpedanz zwischen einem ersten endexspiratorischen Lungenvolumen der Abfolge von positiven endexspiratorischen Drücken und einem zweiten, nachfolgenden endexspiratorischen Lungenvolumen der Abfolge von positiven endexspiratorischen Drücken darstellt;
- Bestimmen eines zweiten Index, der eine Änderung einer Sehnen-Compliance der Lunge des Patienten zwischen dem ersten endexspiratorischen Lungenvolumen und dem zweiten, nachfolgenden endexspiratorischen Lungenvolumen darstellt; und
- basierend auf dem ersten und dem zweiten Index, Bestimmen eines potenziellen Lungenrekrutierungswert für den Patienten.

2. System nach Anspruch 1, wobei das Bestimmen eines potentiellen Lungenrekrutierungswerts für den Patienten ein Zuweisen einer relativen Gewichtung zu jedem des ersten Index und des zweiten Index umfasst.

**3.** System nach Anspruch 2, ferner umfassend Anweisungen, die, wenn sie durch den mindestens einen Prozessor ausgeführt werden, den mindestens einen Prozessor veranlassen, dem ersten Index und dem zweiten Index basierend auf mindestens einem von Alter, Körpermasseindex oder Geschlecht relative Gewichte zuzuweisen.

**4.** System nach Anspruch 1, ferner umfassend Anweisungen, die, wenn sie durch den mindestens einen Prozessor ausgeführt werden, den mindestens einen Prozessor veranlassen, eine Änderung der Sehnen-Compliance der Lunge zu bestimmen, umfassend:

Bestimmen einer Compliance, die durch jedes Pixel eines ersten elektrischen Impedanztomographiebilds dargestellt wird, das das erste endexspiratorische Lungenvolumen darstellt; und
Bestimmen einer Compliance, die durch jedes Pixel eines zweiten elektrischen Impedanztomographiebilds dargestellt wird, das das zweite, nachfolgende endexspiratorische Lungenvolumen darstellt.

**5.** System nach Anspruch 4, wobei das Bestimmen einer Compliance, die durch jedes Pixel dargestellt wird, durch Dividieren einer Luftmenge, die während eines Atemzyklus in mindestens eine Lunge eintritt, durch eine Differenz zwischen einem inspiratorischen Druck bei Nullfluss und einem positiven endexspiratorischen Druck bestimmt wird.

**6.** System nach Anspruch 1, ferner umfassend Anweisungen, die, wenn sie durch den mindestens einen Prozessor ausgeführt werden, den mindestens einen Prozessor veranlassen, den Patienten entweder als einen Responder oder als einen Nicht-Responder zu klassifizieren, basierend darauf, ob der bestimmte potentielle Lungenrekrutierungswert für den Patienten einen Schwellenwert erreicht oder überschreitet.

**7.** System nach Anspruch 4, ferner umfassend ein Beatmungssystem und ein System der elektrischen Impedanztomographie, die mit dem mindestens einen Prozessor wirkgekoppelt sind.

## Revendications

**1.** Système permettant de déterminer une valeur potentielle de recrutement pulmonaire pour un patient, le système comprenant :

un dispositif de tomographie par impédance électrique (404) ;
au moins un processeur (428) ; et
au moins un support de stockage non transitoire lisible par ordinateur (430) sur lequel sont stockées des instructions qui, lorsqu'elles sont exécutées par l'au moins un processeur, amènent l'au moins un processeur à :

- en réponse à une séquence de pressions expiratoires finales positives appliquées à un patient, amener une impédance pulmonaire expiratoire finale à être mesurée par le dispositif de tomographie par impédance électrique à chaque pression expiratoire finale positive de la séquence de pressions expiratoires finales positives ;
- déterminer un premier indice représentant un changement d'impédance pulmonaire expiratoire finale entre un premier volume pulmonaire expiratoire final de la séquence de pressions expiratoires finales positives et un second volume pulmonaire expiratoire final ultérieur de la séquence de pressions expiratoires finales positives ;
- déterminer un second indice représentant un changement de compliance de corde des poumons du patient entre le premier volume pulmonaire expiratoire final et le second volume pulmonaire expiratoire final ultérieur ; et
- sur la base du premier indice et du second indice, déterminer une valeur potentielle de recrutement pulmonaire pour le patient.

**2.** Système selon la revendication 1, dans lequel la détermination d'une valeur potentielle de recrutement pulmonaire pour le patient comprend l'attribution d'un poids relatif à chacun du premier indice et du second indice.

**3.** Système selon la revendication 2, comprenant en outre des instructions qui, lorsqu'elles sont exécutées par l'au moins un processeur, amènent l'au moins un processeur à attribuer les poids relatifs au premier indice et au second indice en fonction d'au moins l'un parmi l'âge, l'indice de masse corporelle ou le sexe.

4.  Système selon la revendication 1, comprenant en outre des instructions qui, lorsqu'elles sont exécutées par l'au moins un processeur, amènent l'au moins un processeur à déterminer un changement de compliance de corde des poumons, comprenant :

    la détermination d'une compliance représentée par chaque pixel d'une première image de tomographie par impédance électrique représentant le premier volume pulmonaire expiratoire final ; et
    la détermination d'une compliance représentée par chaque pixel d'une seconde image de tomographie par impédance électrique représentant le second volume pulmonaire expiratoire final ultérieur.

5.  Système selon la revendication 4, dans lequel la détermination d'une compliance représentée par chaque pixel est déterminée en divisant une quantité d'air entrant dans l'au moins un poumon au cours d'un cycle respiratoire par une différence entre une pression inspiratoire à débit nul et une pression expiratoire finale positive.

6.  Système selon la revendication 1, comprenant en outre des instructions qui, lorsqu'elles sont exécutées par l'au moins un processeur, amènent l'au moins un processeur à classer le patient comme répondeur ou non-répondeur selon que la valeur potentielle de recrutement pulmonaire déterminée pour le patient atteint ou dépasse une valeur seuil.

7.  Système selon la revendication 4, comprenant en outre un système de ventilation et un système de tomographie par impédance électrique couplés de manière fonctionnelle à l'au moins un processeur.

**FIG. 1**

**FIG. 2**

300

320

326

MEMORY DEVICE 328

322

INPUT DEVICES

PROCESSOR

ELECTRONIC DISPLAY 324

ELECTRODE BELT 310

**FIG. 3**

FIG. 4

_500_

| APPLYING A SEQUENCE OF PEEPS TO LUNGS | ~ 505 |

| MEASURING AN END EXPIRATORY LUNG IMPEDANCE OF THE LUNGS | ~ 510 |

| DETERMINING CHANGES IN EELZ BETWEEN APPLIED PEEPS | ~ 515 |

| DETERMINING A COMPLIANCE OF THE LUNGS | ~ 520 |

| DETERMINE A FIRST INDEX REPRESENTING A CHANGE IN EELZ | ~ 525 |

| DETERMINE A CHANGE IN COMPLIANCE BETWEEN APPLIED PEEPS | ~ 530 |

| DETERMINE A SECOND INDEX REPRESENTING THE CHANGE IN COMPLIANCE | ~ 530a |

| DETERMINING AN AMOUNT OF ALVEOLI OF THE LUNGS RECRUITED BY APPLYING THE SEQUENCE OF PEEPS | ~ 535 |

| NORMALIZING THE FIRST AND SECOND INDEXES | ~ 540 |

| WEIGHTING THE FIRST AND SECOND INDEXES | ~ 545 |

| DETERMINING A POTENTIAL LUNG RECRUITMENT VALUE OF THE LUNG | ~ 550 |

FIG. 6A

FIG. 6B

FIG. 7

FIG. 8

$$V = V_{MAX} \, ^*(1\text{-}e^{\text{-}kp})$$

902

$$V = V_{MAX/2} \, ^*(1\text{-}e^{\text{-}kp})$$

901

Volume

Airway Pressure

Invariant property along the curve: $C_{10}/C_{20} = C_{10}/C_{20}$

FIG. 9

*Invariant property along the curve:* $C_p = C_{ZEEP} * e^{-kP}$

FIG. 10

**FIG. 11**

**FIG. 12**

FIG. 13

Low potential
for lung recruitment

% units open at PEEP10 = 88%

% units open at PEEP40 = 82%

EP 3 799 574 B1

FIG. 14

High potential
for lung recruitment

% units open at PEEP5 = 21%

% units open at PEEP40 = 89%

1302b

1303b

1304b

EP 3 799 574 B1

FIG. 15

**EP 3 799 574 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120010520

- EP 2228009 A

**Non-patent literature cited in the description**

- **KNUDSON RJ ; KALTENBORN WT**. Evaluation of lung elastic recoil by exponential curve analysis. *Respir Physiol.*, October 1981, vol. 46 (1), 29-42